# NEUE EUROPÄISCHE PATENTSCHRIFT

(11) **EP 0 303 284 B2**
(45) Veröffentlichungstag und Bekanntmachung des Hinweises auf die Entscheidung über den Einspruch: **03.05.2000**
(45) Hinweis auf die Patenterteilung: 17.06.1992
(21) Anmeldenummer: 88113141.1
(22) Anmeldetag: 12.08.1988
(51) Int. Cl.: G01N 33/543, G01N 33/78, G01N 33/531

(54) **Immunologisches Bestimmungsverfahren zur Bestimmung von Hapteneigenschaften aufweisenden freien Substanzen**
Immunological method for the determination of free haptenic substances
Procédé immunologique pour déterminer des substances hapténiques libres

(30) Priorität: 14.08.1987 DE 3727238
(43) Veröffentlichungstag der Anmeldung: 15.02.1989
(73) Patentinhaber: B.R.A.H.M.S Diagnostica GmbH, D-12099 Berlin (DE)
(72) Erfinder: Gadow, André, Dr., D-6380 Bad Homburg 6 (DE); Hantke, Uwe, D-1000 Berlin 27 (DE); Thoma, Rudy, D-1000 Berlin 42 (DE); Rokos, Hartmut, Dr., D-1000 Berlin 37 (DE)
(74) Vertreter: Andrae, Steffen, Dr.

(56) Entgegenhaltungen:
- EP-A- 89 806
- EP-A- 143 274
- EP-A- 182 385
- EP-A- 246 152
- EP-A- 254 929
- EP-A- 257 352
- EP-A- 0 324 540
- WO-A-83/03306
- WO-A-85/00226
- US-A- 4 434 236
- J. Clinical Immunoassay 7(2), 163-180 (1984)
- Analytical Biochemistry 107, 220-239 (1980); Munson & Rodbard

## Beschreibung

Die Erfindung betrifft ein immunologisches Bestimmungsverfahren zur Bestimmung von Hapteneigenschaften aufweisenden freien Substanzen, insbesondere von Hormonen, Steroiden, Drogen oder deren Metaboliten, Vitaminen oder Toxinen, in biologischen Flüssigkeiten in Gegenwart von einem oder mehreren physiologischen Bindepartnern für die zu bestimmenden Substanzen. Die Gesamtmenge derartiger Substanzen in der zu untersuchenden biologischen Flüssigkeit verteilt sich dabei auf einen freien und einen gebundenen Anteil. Der gebundene Anteil ist dabei an eines oder mehrere physiologische Bindeproteine oder ähnliche Bindungspartner für diese Substanzen gebunden, die in der Lage sind, die jeweilige Substanz mehr oder weniger spezifisch mit einer definierten Affinität zu binden.

Die gebundenen und ungebundenen Anteile stehen miteinander im Gleichgewicht, wobei bei den derzeit gültigen Theorien davon ausgegangen wird, daß die nichtgebundenen Anteile, d. h. die freien Substanzen, die physiologisch wirksame Komponente darstellen, während die gebundenen Anteile eine Art Reservoir zur Bereitstellung dieser Substanz bilden. Man kennt darüberhinaus auch die Bindung an sog. Transportproteine, die für die Verteilung der Substanzen im Organismus und deren Transport zum Wirkort dienen.

In den letzten Jahren hat sich die Überzeugung durchgesetzt, daß der Bestimmung der freien Substanzen, d. h. der physiologisch wirksamen Anteile einer Substanz, in der klinischen Diagnostik eine höhere Wertigkeit zukommt als der gleichzeitigen Bestimmung gebundener und ungebundener Anteile. In einem Artikel von R. P. Ekins mit dem Titel "The direct immunoassay of free (non-protein bound) hormones in body fluids" in: Immunoassays For Clinical Chemistry, herausgegeben von W.N. Hunter und J.E.T. Corrie,Churchill Livingstone, 2. Aufl. (1983), Seite 319 bis 339 werden die Gründe für eine Bestimmung der freien Substanzen anhand von Modellvorstellungen erläutert, und es wird außerdem der Versuch einer Auflistung verschiedener Typen von immunologischen Bestimmungsverfahren unternommen, die für eine Bestimmung freier Substanzen geeignet sein könnten.

Die europäischen Patente 26103 und 73865 sowie das deutsche Patent 34 15 818 beschreiben immunologische Bestimmungsverfahren für freie Substanzen, die bis zur Praxisreife entwickelt wurden.

Da die Ermittlung des physiologischen Schilddrüsenstatus über die Bestimmung der freien Schilddrüsenhormone Thyroxin (T₄) und Trijodthyronin (T₃) medizinisch von besonderer Bedeutung ist, betreffen die genannten Patente vorrangig derartige Bestimmungsverfahren bzw. diskutieren die Anwendung der beschriebenen Grundverfahren für diesen Spezialfall. Auch das in der vorliegenden Anmeldung beschriebene immunologische Bestimmungsverfahren hat seine vorrangige Bedeutung im Rahmen der Ermittlung des physiologischen Schilddrüsenstatus.

Das im lebenden menschlichen Organismus zirkulierende T₄ verteilt sich auf die Bindeproteine Albumin (ca. 10 %), Thyroxin-bindendes Präalbumin (TBPA, ca. 30 %) und Thyroxinbindendes Globulin (TBG, ca. 60 %). Lediglich 0,01 bis 0,03 % liegen als physiologisch wirksames freies T₄ (FT₄) vor. Als Normalbereich ergibt sich dabei für das FT₄ ein Konzentrationsbereich von ca. 8 bis 20 pg/ml Körperflüssigkeit.

Für die Bestimmung von FT₄ stellt die Gleichgewichtsdialyse von Serum derzeit die anerkannte Referenzmethode dar (vgl. Clin. Invest. 45 (1966), Seite 153 bis 163). Hierbei wird dem Serum radioaktives T₄ zugesetzt oder der T₄-Gehalt im Dialysat direkt in einem T₄ Radioimmunoassay ermittelt. Für die klinische Routinediagnostik ist dieses Verfahren jedoch trotz seiner hohen Zuverlässigkeit aufgrund des unvertretbar hohen Zeitaufwands von ca. 20 h pro Bestimmung nicht geeignet.

Neben Verfahren, die einen mehr orientierenden Charakter aufweisen und in Einzelfällen zu erheblichen Ungenauigkeiten führen, wurden daher für die klinische Routinediagnostik immunologische Bestimmungsverfahren entwickelt, die direkte Schlüsse auf die Konzentration der zu bestimmenden freien Substanzen ermöglichen. Dabei arbeiten die bisher bis zur Praxisreife entwickelten derartigen immunologischen Bestimmungsverfahren, die insbesondere Radioimmunoassays sind, nach dem bekannten Konkurrenzprinzip, bei dem der zu untersuchenden Probe eine markierte Form der zu bestimmenden Substanz zugesetzt wird und anschließend nach einer Umsetzung mit einem geeigneten Antikörper aus dem anhand seiner Markierung ermittelten Anteil der gebundenen Form auf die Konzentration der zu bestimmenden Substanz zurückgeschlossen wird. Um bei einer Bestimmung freier Substanzen, insbesondere von FT₄, Störungen durch Varianzen bei den Bindeproteinen auszuschließen, werden dabei als markierte Formen der zu bestimmenden Substanz (FT₄) bei den bekannten Verfahren sog. T₄-Analog-Tracer verwendet, die aufgrund ihrer chemischen Struktur eine deutlich verminderte Affinität zu den Bindeproteinen aufweisen und dadurch das Gleichgewicht freie Substanz/gebundene Substanz möglichst wenig beeinflussen sollen. Die klinische Wertigkeit und Aussagekraft dieser Verfahren wird, zumindestens für bestimmte Spezialfälle, jedoch von verschiedenen Autoren angezweifelt (Helenius, T.,Liewendahl, K., Clin. Chem. 29(5) (1983), Seite 816-822; Mardell, R., Gamlen, T. R., The Lancet, 24. April 1983, Seite 973 bis 974; Gow.,S.M. et al, Clinica Chimica Acta 152 (1985), Seite 325-333; Chopra, I.J. et al, J. Clin. Endocrin. Met. 51(1) (1980), Seite 135-143 und Herrmann, J. et al, Nucl.-Med. 21(5) (1982), Seite 186-191). Die Methoden versagen zumeist bei Schwerkranken (NTI = non thyroidal illness) aufgrund von zahlreichen Stör- und Einflußgrößen (Katabolite, Proteinverlust, endogen oder unter Heparin-Einfluß freigesetzte Fettsäuren) (vgl. Reiners, Ch., Ärztl. Lab. 31 (1985), Seite 331-344). Zu den Analog-Tracer Verfahren ist auch der von Ito, M. et al in: Clin. Chem. 30(10) (1984), Seite 1682 bis 1685 beschriebene Enzymimmunoassay zur Bestimmung von FT₄ zu rechnen, bei dem als Tracer ein T₄-β-D-Galactosidase Konjugat verwendet wird, das nicht an die Bindeproteine binden soll.

Ein von Weetall et al in Clin. Chem. 28(4) (1982), Seite 666-671 beschriebener Enzymimmunoassay basiert auf der Verteilung eines T₄-Meerrettichperoxidase-Konjugats auf freie und proteingebundene Anteile und einem komplexen mathematischen Modell zur Berechnung des FT₄-Anteils. Die beiden zuletzt genannten Verfahren haben bislang keinen Eingang in die klinische Routinediagnostik gefunden.

Bei allen bisher beschriebenen Verfahren wurden markierte Formen der zu bestimmenden Substanzen, also markierte Antigene verwendet.

Die Verwendung markierter Antikörper zum quantitativen Nachweis von mit diesen Antikörpern bindendenAntigenen wurde jedoch grundsätzlich ebenfalls bereits beschrieben, und zwar erstmals von Miles, L.E.M. und Hales, C.N. in: Nature 219, (1968), Seite 186-189. Die Beschreibung einer Möglichkeit zur immunologischen Bestimmung von kleineren Molekülen wie Steroidhormonen unter Verwendung von Festphasen-gekoppeltem Antigen und markierten Antikörpern findet sich bei Stafford, J.E.H. und Kilgallon, W. in: J. Immunol. Methods 34 (1980), Seite 339-343 unter Verwendung einer radioaktiven Markierung. Eine ähnliche Bestimmung unter Verwendung einer Lumineszenz-Markierung ist beschrieben von Wood, W.G. et al in: J. Clin. Chem. Clin. Biochem. 20(1982), Seite 825-831.

In der bereits eingangs zitierten theoretischen Arbeit von Ekins wird auf Seite 330 unter Bezugnahme auf das in Fig. 8.1.J beschriebene Schema eine weitere Möglichkeit zur Ausgestaltung eines immunologischen Bestimmungsverfahrens zur Bestimmung von Hapteneigenschaften aufweisenden freien Substanzen in biologischen Flüssigkeiten in Gegenwart von einem oder mehreren physiologischen Bindeproteinen für die zu bestimmenden Substanzen diskutiert, bei dem die biologische Flüssigkeit umgesetzt wird mit einer definierten Menge eines gegen die zu bestimmende Substanz spezifischen Antikörpers oder eines Antikörpergemischs mit einem detektierbaren Markierungsanteil sowie ferner einem Überschuß der zu bestimmenden Substanz oder eines Derivats dieser Substanz in durch Bindung an eine feste Phase immobilisierter Form, wobei man anschließend die feste Phase von der flüssigen Phase trennt und den Gehalt des markierten Antikörpers in der flüssigen und/oder festen Phase durch Messung der Markierung feststellt und dann durch rechnerische Auswertung der erhaltenen Meßergebnisse den Gehalt der zu bestimmenden freien Substanzen der biologischen Probe ermittelt. Damit die zu diesem Verfahren angestellten Überlegungen eine Gültigkeit aufweisen, wird bezüglich der Eigenschaften des festphasen-gebundenen Antigens eine 100 %ige Kreuzreaktivität zum eingesetzten markierten Antikörper oder Antikörpergemisch im Vergleich mit dem in freier Form vorliegenden Antigen vorausgesetzt. Somit sollte der markierte Antikörper vergleichbar große Bindungsaffinitäten zum freien und festphasen-gebundenen Antigen aufweisen, um eine Bestimmung nach diesem Verfahren zu ermöglichen.Die praktisch erfolgreiche Entwicklung eines derartigen Bestimmungsverfahrens ist jedoch noch nicht berichtet worden.

In der DE-OS 34 42 817 wird eine Abwandlung des eben beschriebenen Verfahrensprinzips zur quantitativen Bestimmung von FT₄ beschrieben, bei dem die Probe zuerst höchstens 10 min mit einem 10 bis 2000fachen Unterschuß an markiertem anti- T₄-Antikörper, bezogen auf die molare Menge des Gesamt-T₄, inkubiert wird. Anschließend bringt man sofort mit überschüssigem immobilisiertem T₄ zusammen und inkubiert erneut für mindestens 1 min. Danach werden die Phasen getrennt, und in einer der Phasen wird die Markierung gemessen. Dieses Verfahren gemäß DE-OS 34 42 817 ist ein typisches "kinetisches" Verfahren, das offensichtlich von der Annahme ausgeht, daß während der kurzen ersten Inkubation mit dem markierten anti-T₄-Antikörper zuerst nur die in freier Form vorliegenden Mengen von T₄ erfaßt werden, ohne daß es zu einer die Meßergebnisse verfälschenden langsamen Nacheinstellung des Gleichgewichts unter Freisetzung von vorher gebundenem T₄ kommt. Da die Inkubationsdauer der Vorinkubation das Testergebnis durch die bekannte rasche Gleichgewichtseinstellung zwischen freiem T₄ und gebundenem T₄ beeinflussen kann, sollte dieser Test wie alle ähnlichen zweistufigen Tests den Nachteil aufweisen, gegenüber abweichenden Testbedingungen anfällig zu sein, was die Anwendung des entsprechenden Verfahrens in der klinischen Praxis erschwert.

Aus der US-A-4 434 236 ist ein Verfahren zur Bestimmung eines Analyten, insbesondere von Digoxin bekannt, bei dem so vorgegangen wird, daß zuerst an ein immobilisiertes Analytanaloges (Oubain) ein Überschuß eines markierten Antikörpers gebunden wird. Der gebildete immobilisierte Immunkomplex wird anschließend mit der zu untersuchenden Probe umgesetzt. Aufgrund der höheren Affinität des zu bestimmenden Analyten (Digoxin) gegenüber dem gebundenen markierten Antikörper wird dieser von dem schwächer bindenden Analytanalogen verdrängt und kann mit dem zu bestimmenden Analyten einen löslichen Immunkomplex bilden, der bestimmt werden kann. Bei diesem Verfahren geht es um die Bestimmung der Gesamtkonzentration des Analyten, nicht jedoch um die Bestimmung des freien Anteils eines Analyten, der in der zu untersuchenden Probe gleichzeitig in an einen physiologischen Bindepartner (ein Bindeprotein) gebundener Form vorliegt.

Der Erfindung liegt die Aufgabe zugrunde, ein für die klinische Routinediagnostik geeignetes, einfach durchzuführendes und von der klinischen Aussage her relevantes Verfahren zur Bestimmung des freien Anteils einer Substanz in einer biologischen Flüssigkeit neben dem gleichzeitig vorliegenden an einen physiologischen Binderpartnern gebundenen Anteil dieser Substanz zu schaffen, das eine optimale Empfindlichkeit aufweist und auf der Seite der Hersteller der für das Bestimmungsverfahren benötigten Materialien und Substanzen eine optimale Qualitätskontrolle ermöglicht.

Diese Aufgabe wird gelöst durch ein immunologisches Bestimmungsverfahren zur Bestimmung von Hapteneigenschaften aufweisenden freien Substanzen, insbesondere von Hormonen, Steroiden, Drogen oder deren Metaboliten, Vitaminen oder Toxinen, in biologischen Flüssigkeiten in Gegenwart von einem oder mehreren physiologischen Bindepartnern für die zu bestimmenden Substanzen, bei dem die biologische Flüssigkeit umgesetzt wird mit
a) einer definierten Menge eines gegen die zu bestimmende Substanz spezifischen Antikörpers oder eines Antikörpergemischs mit einem detektierbaren Markierungsanteil und
b) einem Überschuß der zu bestimmenden Substanz oder eines Derivats dieser Substanz in durch Bindung an eine feste Phase immobilisierter Form,
und bei dem man anschließend die feste Phase von der flüssigen Phase trennt und den Gehalt des markierten Antikörpers in der flüssigen und/oder festen Phase durch Messung der Markierung feststellt und dann durch rechnerische Auswertung der erhaltenen Meßergebnisse unter Verwendung von Eichkurven den Gehalt der zu bestimmenden freien Substanz in der biologischen Probe ermittelt, dadurch gekennzeichnet, daß die immobilisierte Form der Substanz oder ihres Derivats so ausgewählt wurde, daß die Affinität des spezifischen markierten Antikörpers oder Antikörpergemischs gegenüber der immobilisierten Form weniger als 28 % der Affinität dieses Antikörpers oder Antikörpergemischs gegenüber der Substanz beträgt, deren freier Anteil in der biologischen Flüssigkeit bestimmt werden soll ("Kreuzreaktivität < 50 %").

Vorteilhafte Ausgestaltungen sind den Unteransprüchen zu entnehmen. Der vorliegenden Erfindung liegt die überraschende Erkenntnis zugrunde, daß für die Entwicklung eines immunologischen Bestimmungsverfahrens auf der Basis eines an sich bekannten Grundverfahrens, wie es z.B. von Ekins (a.a.O) beschrieben wurde, die festphasen-gebundenen Antigene eine stark verminderte Kreuzreaktivität von unter 28 %, vorzugsweise im Bereich von 8 bis 25 %, im Vergleich zum zu bestimmenden freien Antigen aufweisen müssen, wenn ein Bestimmungsverfahren mit einer für praktische Zwecke ausreichend hohen Empfindlichkeit und Reproduzierbarkeit erhalten werden soll. Es zeigte sich überraschend, daß höhere Kreuzreaktivitäten, die im Bereich der bei Ekins (a.a.O) vorausgesetzten 100 %igen Kreuzreaktivität oder darüber liegen, zu deutlich verfälschten Ergebnissen führen und daß der Meßbereich dann außerhalb des klinisch relevanten Konzentrationsbereichs für die freie Substanz liegt. Ferner zeigte sich, daß bei der Einhaltung von Kreuzreaktivitäten im angegebenen Bereich die bei dem Verfahren gemäß DE-OS 34 42 817 erforderliche Vorinkubation überflüssig ist und das Verfahren einstufig durchgeführt werden kann, indem die biologische Flüssigkeit im wesentlichen gleichzeitig mit dem markierten Antikörper und dem im Überschuß vorliegenden immobilisierten Antigen umgesetzt werden kann.

Diese Möglichkeit einer einstufigen Arbeitsweise stellt einen erheblichen Vorteil dar. Durch die Auswahl geeigneter Antikörper oder Antikörpergemische bestimmter Affinität wird bei der einstufigen Arbeitsweise nicht oder nur unwesentlich in das Gleichgewicht freie Substanz/gebundene Substanz eingegriffen, so daß bedingt durch die unterschiedlichen Kreuzreaktivitäten von freier Substanz bzw. immobilisierter Substanz zum markierten Antikörper lediglich die Bindungskinetik des markierten Antikörpers an das immobilisierte Antigen zu berücksichtigen ist. Dies wirkt sich jedoch bei unterschiedlichen Inkubationszeiten nur auf die Bindungsverhältnisse und nicht auf eine Verschiebung des Gleichgewichts freie Substanz/gebundene Substanz aus.

Es ist somit bevorzugt, das erfindungsgemäße Verfahren als einstufiges Verfahren ohne Vorinkubation durchzuführen, was eine wesentliche Vereinfachung bedeutet.

Obwohl das erfindungsgemäße Verfahren generell zur Bestimmung von Hapteneigenschaften aufweisenden freien Substanzen, insbesondere von Hormonen, Steroiden, Drogen oder deren Metaboliten, Vitaminen oder Toxinen, in biologischen Flüssigkeiten geeignet sein dürfte, hat es besondere Bedeutung im Zusammenhang mit der Bestimmung von FT₄ und FT₃.

Das erfindungsgemäße Verfahren weist ferner alle bekannten Vorteile von Bestimmungsverfahren unter Verwendung eines durch Bindung an eine feste Phase immobilisierten Umsetzungspartners auf. Durch die Immobilisation an einer festen Phase werden die erforderlichen Waschschritte deutlich vereinfacht, und die Präzision der Bestimmung wird verbessert. Als Festphasen, an die die immobilisierte Form der zu bestimmenden Substanz gebunden ist, eignen sich alle an sich bekannten inerten Trägermaterialien mit ausreichend stabilen Bindungseigenschaften und einer ausreichend hohen Bindungskapazität, wozu Kunststoffe wie Polystyrol, Polyethylen und Teflon oder Glas zu zählen sind. Geeignete Festphasen sind auch in der US-PS 4 657 873 und in einer Publikation von Wood, W.G. und Gadow, A. in: J. Clin. Chem. Clin. Biochem. 21 (1983) Seite 789-797 beschrieben. Die Markierung der verwendeten Antikörper erfolgt nach bekannten Verfahren mit an sich für eine Markierung geeigneten detektierbaren Markierungsanteilen. Im Rahmen der vorliegenden Erfindung sind Markierungen mit relativ kleinen Markern, die die Reaktivität der Antikörper relativ wenig beeinflussen, bevorzugt. Das sind insbesondere Radioisotope, insbesondere Jodisotope, und Luminogene. Unter der Voraussetzung, daß die Markierungsanteile eine Einhaltung der geforderten Kreuzreaktivitäten gegenüber einem geeigneten Antikörper ermöglichen, sind als Marker jedoch z. B. auch geeignet Enzyme, Substrate, fluoreszierende Markierungen, phosphoreszierende Markierungen, Biotin (nachweisbar über markiertes Avidin) oder Kofaktoren. Auch indirekte Markierungsverfahren können angewandt werden, bei denen der Marker vor der Messung wieder vom Antikörper abgelöst wird, sowie alle aufgrund einer visuellen, physikalischen oder chemischen Reaktion quantifizierbar nachweisbaren Substanzen.

Als Antikörper sind für das erfindungsgemäße Verfahren alle als für derartige Verfahren geeignet bekannten Antikörper geeignet. Derartige Antikörper weisen vorzugsweise eine Affinität gegenüber T₄ bzw. T₃ auf, die höchstens der Affinität von T₄ bzw. T₃ gegenüber den physiologischen Bindeproteinen entspricht. Derartige anti-T₄ (bzw. T₃)-Antikörper weisen üblicherweise eine Affinitätskonstante von 10¹⁰ l/mol oder weniger auf.

Bei dem erfindungsgemäßen Verfahren wird die immobilisierte Substanz oder ihr Derivat vorzugsweise in Form eines Konjugats mit einer Trägersubstanz an der Festphase immobilisiert. Als Trägerkomponente kommen dabei hochmolekulare Substanzen wie Proteine, Polypeptide oder Polysaccharide in Frage, gegenüber denen der markierte Antikörper bzw. das markierte Antikörpergemisch eine Kreuzreaktivität von weniger als 0,5 % aufweisen. Geeignet sind bevorzugt solche Trägerkomponenten, die nicht mit der Trägerkomponente identisch sind, an die die zu bestimmende Substanz für die Produktion der eingesetzten Antikörper gebunden war.

Es hat sich dabei gezeigt, daß die Kreuzreaktivitäten eines bestimmten Konjugats, auch wenn es aus identischen Ausgangsmaterialien unter identischen Bedingungen hergestellt wurde, stark streuen können. Scheinbar identische Konjugate, insbesondere in immobilisierter Form, können daher sehr unterschiedliche Kreuzreaktivitäten aufweisen. Zur Bestimmung einer Eignung für das erfindungsgemäße Verfahren sind daher die Kreuzreaktivitäten einer bestimmten Charge gegenüber dem zu verwendenden Antikörper jeweils zu bestimmen. Durch die Bestimmung der Kreuzreaktivität ist dann eine eindeutige Aussage über die Einsetzbarkeit des betreffenden Konjugats im erfindungsgemäßen Verfahren möglich. Diese Verhältnisse unterscheiden sich deutlich von den Verhältnissen, die im Falle der direkten Verwendung der zu bestimmenden Substanz oder ihres Derivats in immobilisierter Form oder in Lösung vorliegen, wo die Kreuzreaktivität gegenüber einem bestimmten Antikörper nur von der Art der Substanz selbst anhängt.

Die Inkubationsbedingungen für die Durchführung des erfindungsgemäßen Verfahrens hängen innerhalb gewisser Grenzen von den jeweiligen verwendeten Antikörpern, auch unter Berücksichtigung der Beeinflussung ihrer Bindungseigenschaften durch die verwendete Markierung, sowie von den genauen Kreuzreaktivitäten innerhalb des durch die vorliegende Erfindung festgelegten Bereichs ab. Geeignete Inkubationsbedingungen umfassen Inkubationstemperaturen von 17 bis 37°C und Inkubationszeiten von 30 min bis 3 h. Bevorzugte Inkubationsbedingungen sind die in den Beispielen angewandten Inkubationsbedingungen, die eine zweistündige Inkubation (± 10 min) bei 22°C vorsehen, wobei geschüttelt wird, vorzugsweise auf einem Horizontalschüttler.

Nachfolgend wird die Bedeutung der verminderten Kreuzreaktivität der festphasen-gebundenen Substanz bzw. ihres Derivats für die Qualität des erhaltenen Testverfahrens anhand von Beispielen näher erläutert, die die im Rahmen der vorliegenden Erfindung bevorzugte Bestimmung von FT₃ und FT₄ betreffen.

### Herstellung von für die Immobilisierung verwendeten T₄-Derivaten

### 1. L-Tetrajodthyroninethylester (L-T₄ OEt)

L-T₄OEt wurde nach der modifizierten Methode von Clayton, J.C. und Hems, B.A., J.Org.Chem., 1950, Seite 840-843 hergestellt.

### 2. N-Trifluoracetylthyroxin (TFAT₄)

Die Titelverbindung wurde nach der modifizierten Methode von Schroeder et al in: Methods in Enzymology, Vol. 57, Bioluminescence and Chemiluminescence, New York, Academic Press 1978, Seite 424-445 hergestellt.

Dazu wurden 5 g L-Tetrajodthyronin in 60 ml absolutem Ethylacetat gelöst, und es wurden 11,5 ml Trifluoressigsäure sowie 1,9 ml Trifluoressigsäureanhydrid zugesetzt. Die Mischung wurde 1 h bei 0°C gerührt.

Anschließend ließ man die Reaktionsmischung auf Raumtemperatur erwärmen. Es wurden 200 ml Wasser zum Reaktionsansatz zugefügt, und die resultierende Lösung wurde mit Natriumchlorid gesättigt. Die organische Phase wurde abgetrennt, mit einer gesättigten Natriumchloridlösung gewaschen und über trockenem Magnesiumsulfat getrocknet. Die getrocknete Phase wurde abfiltriert und bis zur Trockne eingeengt. Der saubere Rückstand wurde ohne weitere Aufreinigung für die weiteren Synthesen eingesetzt.
Ausbeute: 4,8 g

### Analysendaten:

| Elementaranalyse | | | |
|---|---|---|---|
| Berechnet: | C 23,39 % | H 1,15 % | N 1,60 % |
| Gefunden: | 23,19 % | 1,12 % | 1,63 % |

### Herstellung von Konjugaten von T₄ oder T₄-Derivaten

### Konjugat 1: IgG-L-T₄OEt-Konjugat (Kopplungsstelle NH₂)

Das Konjugat wurde nach der aktiv-Ester-Methode hergestellt.

80,5 mg L-T₄OEt und 11 mg Bernsteinsäureanhydrid wurden in 2 ml trockenem und aminfreiem DMF gelöst und über Nacht bei Raumtemperatur gerührt. Danach wurden 12,6 mg N-Hydroxysuccinimid und 22,6 mg N,N'-Dicyclohexylcarbodiimid zum Reaktionsansatz zugesetzt und 1 h bei Raumtemperatur gerührt.

Der Ansatz des aktiven Esters wurde ohne Weitere Aufreinigung zur Konjugatherstellung eingesetzt.

Dazu wurden 100 mg Kaninchen-IgG (SIGMA, München) in 20 ml Wasser gelöst. 200 µl des aktiven Esters wurden in 800 µl trockenem und aminfreiem DMF verdünnt und zu der wäßrigen Lösung des Kaninchen-IgG gegeben. Der Reaktionsansatz wurde nach ca. 12 h durch Ultrafiltration gereinigt.
Ausbeute: 85 mg
Die UV-spektroskopisch ermittelte Einbaurate von L-T₄OEt pro Mol IgG betrug 4,2.

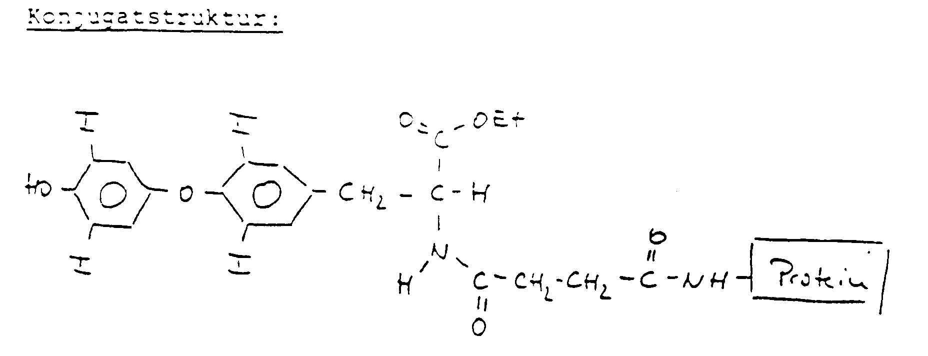

### Konjugat 2: IgG-L-T₄-Konjugat (Kopplungsstelle NH₂ und COOH)

Das Konjugat wurde mittels der Carbodiimid-Methode hergestellt.

1 g Kaninchen-IgG (SIGMA, München) wurde in 100 ml bidestilliertem Wasser gelöst und auf 10 bis 15°C temperiert.

200 mg L-Tetrajodthyronin (Henning Berlin, Berlin) wurden in 5 ml einer alkalischen 1 : 1 Mischung aus trockenem, aminfreiem DMF und Methanol gelöst.

Zu der wäßrigen IgG-Lösung wurden unter Rühren in Abständen von jeweils 1,5 Stunden je 1,25 ml der L-Tetrajodthyronin-Lösung und 100 mg festes 1-Ethyl-2-(3-dimethylaminopropyl)-carbodiimid (EDC) gegeben und der pH-Wert zwischen 5 und 6 gehalten. Nach der letzten Zugabe wurde der Ansatz über Nacht bei 4°C belassen und anschließend durch Ultrafiltration aufgereinigt.
Ausbeute: 860 mg
Die UV-spektroskopisch ermittelte Einbaurate von L-T₄ pro Mol IgG betrug 4,3.

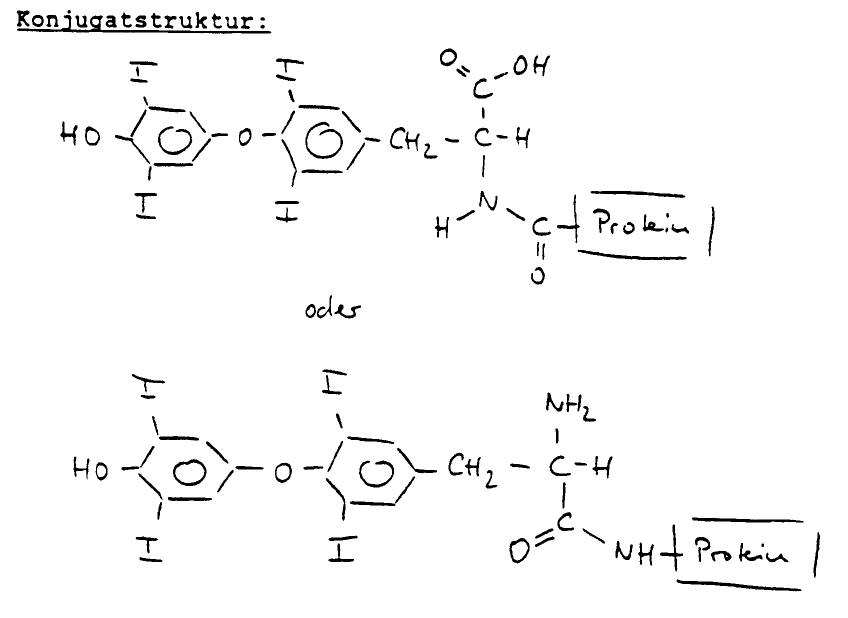

### Konjugat 3: IgG-L-T₄-Konjugat (Kopplungsstelle COOH)

Das Konjugat wurde nach der aktiv-Ester-Methode hergestellt.

43,6 mg N-Trifluoracetylthyroxin, 6,32 mg N-Hydroxysuccinimid und 11,33 mg N,N′-Dicyclohexylcarbodiimid wurden in 1 ml trockenem, aminfreiem DMF gelöst und 1 h bei Raumtemperatur gerührt.

100 mg Kaninchen-IgG (SIGMA, München) wurden in 20 ml bidestilliertem Wasser gelöst.

400 µl des Ansatzes des aktiven Esters wurden mit 600 µl trockenem und aminfreiem DMF verdünnt und zu der wäßrigen IgG-Lösung zugesetzt. Der Reaktionsansatz wurde nach ca. 12stündigem Rühren bei Raumtemperatur unter ammoniakalischen Bedingungen durch Ultrafiltration aufgereinigt, wobei gleichzeitig die Trifluoracetyl-Schutzgruppe abgespalten wurde.
Ausbeute: 94 mg
Die UV-spektroskopisch ermittelte Einbaurate von L-T₄ pro Mol IgG betrug 11,5.

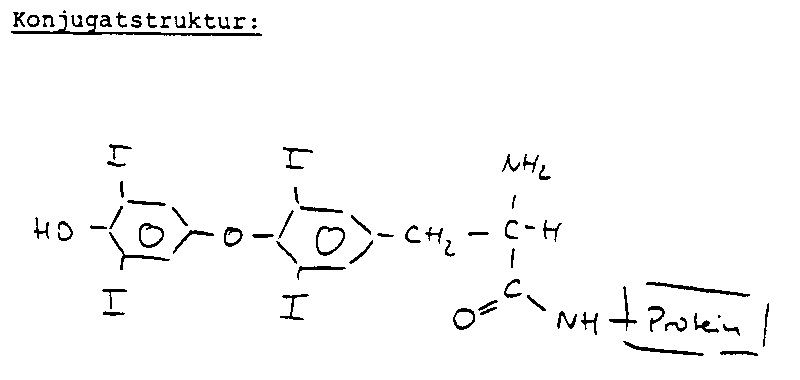

### Konjugat 4: IgG-L-T₄OEt-Konjugat (Kopplungsstelle NH₂)

Das Konjugat wurde mittels der Carbodiimidmethode hergestellt.

10 g Kaninchen-IgG (SIGMA, München) wurden in 1000 ml bidestilliertem Wasser gelöst.

1,25 g L-T₄OEt wurden in 40 ml Methanol (leicht sauer) gelöst und zusammen mit 2,5 g EDC zu der wäßrigen IgG-Lösung gegeben. Die Reaktionsmischung wurde bei Raumtemperatur und gleichbleibendem pH-Wert von etwa 5 unter Lichtausschluß 2 Stunden gerührt und anschließend über Nacht bei 4°C gelagert. Der Ansatz wurde dann mittels Ultrafiltration aufgereinigt.
Ausbeute: 6,8 g
Die UV-spektroskopisch ermittelte Einbaurate von L-T₄OEt pro Mol IgG betrug 1,5.

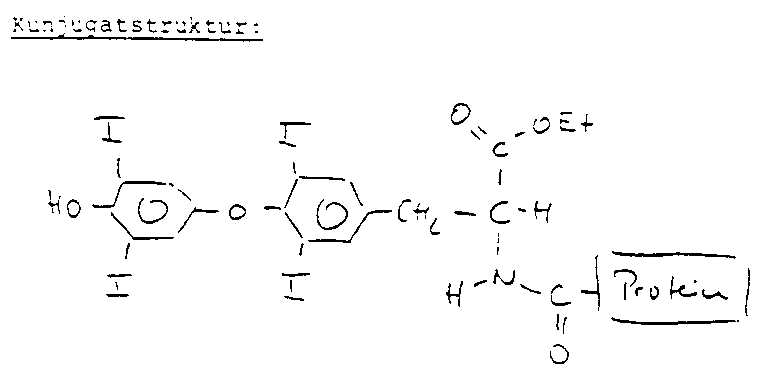

### Konjugat 5: L-T₄-IgG-Konjugat (Kopplungsstelle NH₂ und COOH)

Das Konjugat wurde mittels der Carbodiimidmethode hergestellt.

500 mg L-Tetrajodthyronin (Henning Berlin, Berlin) wurden unter schwach alkalischen Bedingungen in 500 ml bidestilliertem Wasser mit 10 % trockenem und aminfreiem DMF gelöst.

5 g Kaninchen-IgG wurden in 500 ml bidestilliertem Wasser gelöst. Die beiden wäßrigen Lösungen von L-Tetrajodthyronin und Kaninchen-IgG wurden gemischt und in Abständen von etwa 10 min insgesamt 1,9 g EDC in 10 Portionen unter Rühren zugesetzt. Anschließend wurde die Reaktionsmischung über Nacht bei 4°C stehen gelassen und dann durch Ultrafiltration aufgereinigt.
Ausbeute: 2,3 g
Die UV-spektroskopisch ermittelte Einbaurate von L-T₄ pro Mol IgG betrug 3,3.

Die Struktur des erhaltenen Konjugats entsprach der bei Konjugat 2 abgebildeten Struktur.

### Ermittlung der Kreuzreaktivitäten der hergestellten Konjugate gegenüber Antikörpern

Allgemeines Verfahren: Zur Durchführung der Kreuzreaktivitätsversuche wurden die eingesetzten poly- oder monoklonalen Antikörper bzw. Gemische dieser Antikörper kovalent an epoxidgruppenhaltige Mikropartikel mit einer einheitlichen Partikelgröße von 1,055 ± 0,032 µm gebunden. Die Kopplungsmenge der Antikörper/Antikörpergemische betrug jeweils 350 µg gereinigter Antikörper bzw. Antikörpergemisch pro Gramm Mikropartikel. Mögliche nach der Kopplung vorhandene weitere Bindungsstellen wurden mit inerten Substanzen abgesättigt. Die so hergestellten Mikropartikel wurden in 10 ml 0,1 M Phosphatpuffer inkl. 1 mol/l NaCl und 0,05 % Azid, pH 7,2 pro Gramm Mikropartikel aufgenommen.

Als Tracer wurde für die Kreuzreaktivitätsversuche ¹²⁵I-markiertes Thyroxin mit einer spezifischen Aktivität von 6,22 MBq/µg in einer Konzentration von 27,6 mg/l eingesetzt.

Die zu untersuchenden Konjugate sowie das als Bezugssubstanz (zu bestimmende Substanz) dienende Thyroxin (bzw. Trijodthyronin) wurden zur Testdurchführung in einer bindeproteinfreien Puffermatrix aus 20 mmol/l Phosphatpuffer mit 0,2 % Gelatine jeweils frisch angesetzt. Folgende Konzentrationen wurden dabei gewählt:
FT₄: 7,8; 16; 31; 62; 125; 250 und 500 ng L-T₄/ml

Die Konzentrationen der Konjugate wurden wie folgt eingestellt:
Konjugate: 0,01; 0,1; 1; 10 und 100 µg Konjugat/ml.

Für die Kreuzreaktivitätsversuche wurde folgendes Ansatz schema eingehalten:
- 100 µl: Puffer (O-Standard) bzw. Standard
- + 100 µl: ¹²⁵I-markietes Thyroxin (Tracer)
- + 1 ml: Mikropartikelsuspension einer geeigneten Verdünnungsstufe
Dabei wurde die einzusetzende Verdünnung der mit den Antikörpern gekoppelten Mikropartikelsuspension so eingestellt, daß als Bereich der größten Differenzierbarkeit ein Bereich zwischen 30 und 40 ng T₄/ml gefunden wurde.

Der Ansatz wurde 1 h bei 22°C inkubiert.

Eine Zentrifugation bei 2000 g für 10 min und anschließendes Absaugen des Überstandes von den bei der Zentrifugation abgelagerten Mikropartikeln bis zur Trockne (bound-free-Trennung) und die Messung der gebundenen Phase im Gamma-Counter für 60 Sekunden lieferte die Ergebnisse, aus denen die jeweiligen Kreuzreaktivitäten der zu untersuchenden Konjugate nach bekannten Methoden ermittelt wurden.

Die bei Verwendung von Maus-Antikörpern erhaltenen Kreuzreaktivitäten sind in der nachfolgenden Tabelle in der vorletzten Spalte angegeben.

| Konj.-Nr. | L-T₄-Derivat | Kopplungsstelle | Einbaurate (Mol T₄/Mol IgG) | x-Reakt. (%) | 50%-Intercept (pg/ml) |
|---|---|---|---|---|---|
| 1 | L-T₄OEt | NH₂ | 4,2 | 310 | 70 |
| 2 | L-T₄ | NH₂/COOH | 4,3 | 36 | 40 |
| 3 | L-T₄ | COOH | 11,5 | 28 | 17 |
| 4 | L-T₄OEt | NH₂ | 1,5 | 13,5 | 16 |
| 5 | L-T₄ | NH₂/COOH | 3,3 | 9,9 | 15 |

Die Tabelle enthält als letzte Spalte eine Spalte "50 %-Intercept" (pg/ml), der anhand der nachfolgenden Beispiele 1 und 2 ermittelt wurde. Diese Spalte läßt erkennen, ob ein bestimmtes Konjugat in Verbindung mit dem untersuchten Antikörper für eine Verwendung im erfindungsgemäßen Verfahren geeignet war. Wenn der 50 %-Intercept-Wert, der den Bereich die größten Steigung der Standardkurve und damit die größte Differenzierbarkeit geringer Konzentrationsunterschiede wiedergibt, im physiologisch normalen Konzentrationsbereich für FT₄ von 8 bis 20 pg/ml liegt, ist das entsprechende Konjugat für eine Verwendung im erfindungsgemäßen Verfahren sehr gut geeignet.

Es ist zu erkennen, daß diese Bedingungen für solche Konjugate erfüllt sind, deren Kreuzreaktivität unter 28 % (von 9,5 bis 28 %) liegt. Bei einer Kreuzreaktivität von 36 % liegt der 50 %-Intercept bei 40 pg/ml, was bereits eine nennenswerte Verminderung der Empfindlichkeit des Tests bedeutet.

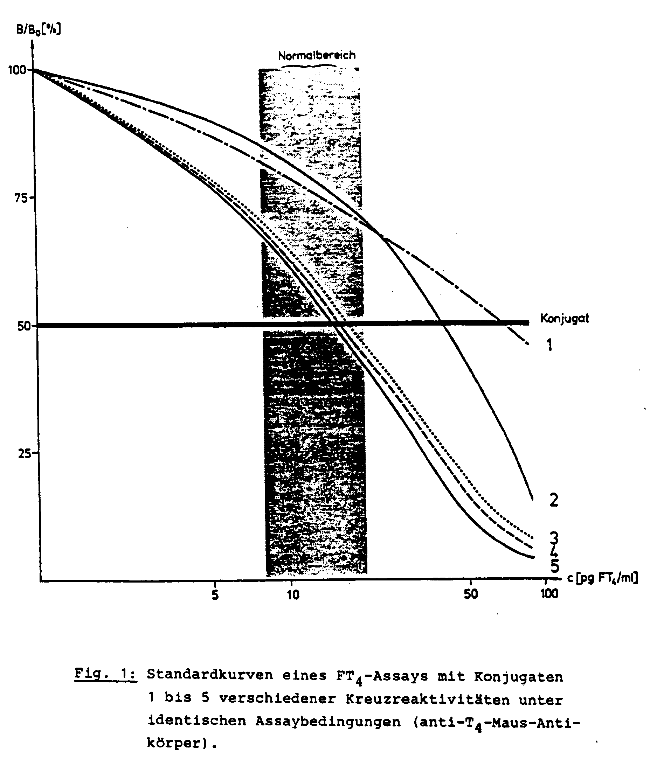

Die in der letzten Spalte der obigen Tabelle wiedergegebenen Werte, die unter identischen Assaybedingungen (Beispiel 1 + 2) ermittelt wurden, können auch der beigefügten Figur 1 entnommen werden.

Das erfindungsgemäße Verfahren, das in der beschriebenen Form auch zur Ermittlung der in der letzten Spalte der Tabelle wiedergegebenen Werte bzw. der in Fig. 1 dargestellten Kurve verwendet wurde, wird nachfolgend anhand von zwei konkreten Ausführungsbeispielen noch näher beschrieben:

### Beispiel 1

### Bestimmungsverfahren für freies Thyroxin (FT₄)

Jeweils 1 µg des zu untersuchenden Konjugats wurde an die Festphase gekoppelt, die im vorliegenden Beispiel ein an sich bekanntes beschichtetes Polystyrolröhrchen war.

Als Antikörper wurde ein monoklonaler, T₄-spezifischer Antikörper aus der Maus verwendet. Der Antikörper war auf bekannte Weise mit ¹²⁵I markiert. Die spezifische Aktivität des resultierenden markierten Antikörpers lag zwischen 25 und 35 KBq/µg Antikörper. Der markierte Antikörper wurde in 0,1 M Phosphatpuffer mit 1 mol/l NaCl und 0,05 % Azid, pH 7,2 aufgenommen. Die Konzentration des markierten Antikörpers lag dabei zwischen 1 und 1,25 µg/l.

Als Standardmaterial wurde eine Humanserum-Matrix mit den folgenden Konzentrationen eingesetzt:
0; 2,8; 5,6; 11,3 ; 22,5; 45 und 90 pg FT₄/ml.

Der Assay wurde wie folgt durchgeführt:

In das Polystyrolröhrchen mit dem zu untersuchenden immobilisierten Konjugat wurden 50 µl Standardmaterial pipettiert und es wurden 500 µl ¹²⁵I-markierter Antikörper (Tracer) zugesetzt.

Die Umsetzungspartner wurden 2 h bei 22°C auf dem Horizontalschüttler inkubiert. Durch Absaugen der Inkubationslösung aus allen Röhrchen wurde die Immunreaktion beendet.

Alle Röhrchen wurden 3mal mit je 4 ml Waschlösung (0,15 mol/NaCl) aufgefüllt und anschließend dekantiert.

Die an die feste Phase gebundene Restaktivität wurde im Gamma-Counter für 60 Sekunden gemessen. Die erhaltenen Ergebnisse wurden durch Datenreduktion nach bekannten Methoden ausgewertet.

### Beispiel 2

### Bestimmung von freiem Thyroxin (FT₄)

Zur Immobilisierung des zu untersuchenden Konjugats wurden wie in Beispiel 1 beschichtete Polystyrolröhrchen eingesetzt.

Als Antikörper diente wieder ein monoklonaler, T₄-spezifischer Antikörper aus der Maus, der jedoch diesmal nicht mit einem Radionuklid sondern mit einem Luminogen markiert war. Das Luminogen war auf an sich bekannte Weise (vgl. US-PS 4 645 646; DE-OS 29 21 781 oder DE-OS 31 32 491) an den Antikörper gekoppelt. Als Luminogen wurde ein cyclisches Diacylhydrazid-Derivat verwendet.

Der mit dem Luminogen markierte Antikörper wurde in 0,1 M Phosphatpuffer mit 1 mol/l NaCl und 0,05 % Azid, pH 7,2 aufgenommen. Die Konzentration des markierten Antikörpers lag dabei zwischen 1 und 1,25 µg/l.

Als Standardmaterial wurde die gleiche Humanserum-Matrix wie in Beispiel 1 verwendet. Auch die Assay-Durchführung entsprach vollständig der von Beispiel 1.

Die Messung der festphasen-gebundenen Restaktivität erfolgte in einem zur Messung von Chemilumineszenz geeigneten Luminometer mit mindestens einer Injektionsmöglichkeit in der Meßposition (Berthold LB 9502 oder Hamilton LUMICON) für 4 s. Das Meßverfahren und die dabei verwendeten Reagenzien sind u. a. in der bereits zitierten US-PS 4 645 646 näher beschrieben.

Eine anschließende Datenreduktion nach bekannten Methoden lieferte die gesuchten Konzentrationen.

In den beiden Verfahrensbeispielen 1 und 2 wurden als Festphasen beschichtete Polystyrolröhrchen eingesetzt. Es können jedoch ohne weiteres als Festphasen auch andere Kunststoffe (z. B. Polypropylen, Nylon, Teflon u. a. geeignete aktivierte Kunststoffe) sowie Glas eingesetzt werden. Die Kopplung der Konjugate erfolgt dabei stets nach einschlägig bekannten Methoden, z. B. adsorbtiv oder kovalent (vgl. Catt, K.,Tregear, G.W., in: Science, 158 (1967), Seite 1570-1572; US-PS 4 657 873 oder Wood, W.G. und Gadow, A. in: J. Clin. Chem. Clin. Biochem. 21 (1983),Seite 789-797.

Anstelle der in den Beispielen 1 und 2 verwendeten Markierungen mit Radionukliden(¹²⁵I) und Luminogenen kann auch auf andere, an sich bekannte Weise mit Enzymen, Substraten, Fluoreszern oder anderen detektierbaren Substanzen markiert werden, wobei dann selbstverständlich die geeigneten Nachweisverfahren zu wählen sind.

Die in die vorliegende Anmeldung aufgenommenen Versuche betreffen alle den Nachweis von FT₄. Versuchsergebnisse, die die Eignung des erfindungsgemäßen Verfahrens in gleicher Weise zur Bestimmung von FT₃ betreffen, liegen ebenfalls vor. Sie bestätigen die Aussagen in der vorliegenden Beschreibung.

## Patentansprüche

1. Immunologisches Bestimmungsverfahren zur Bestimmung von Hapteneigenschaften aufweisenden freien Substanzen, insbesondere von Hormonen, Steroiden, Drogen oder deren Metaboliten, Vitaminen oder Toxinen, in biologischen Flüssigkeiten in Gegenwart von einem oder mehreren physiologischen Bindepartnern für die zu bestimmenden Substanzen, bei dem die biologische Flüssigkeit umgesetzt wird mit
a) einer definierten Menge eines gegen die zu bestimmende Substanz spezifischen Antikörpers oder Antikörpergemischs mit einem detektierbaren Markierungsanteil und
b) einem Überschuß der zu bestimmenden Substanz oder eines Derivats dieser Substanz in durch Bindung an eine feste Phase immobilisierter Form,
und bei dem man anschließend die feste Phase von der flüssigen Phase trennt und den Gehalt des markierten Antikörpers in der flüssigen und/oder festen Phase durch Messung der Markierung feststellt und dann durch rechnerische Auswertung der erhaltenen Meßergebnisse unter Verwendung von Eichkurven den Gehalt der zu bestimmenden freien Substanz in der biologischen Probe ermittelt,
**dadurch gekennzeichnet**, daß die immobilisierte Form der Substanz oder ihres Derivats so ausgewählt wurde, daß die Affinität des spezifischen markierten Antikörpers oder Antikörpergemischs gegenüber der immobilislerten Form weniger als 28 % der Affinität dieses Antikörpers oder Antikörpergemischs gegenüber der Substanz beträgt, deren freier Anteil in der biologischen Flüssigkeit bestimmt werden soll ("Kreuzreaktivität < 28 %").

2. Verfahren nach Anspruch 1, dadurch gekennzeichnet, daß die Kreuzreaktivität im Bereich von 8 bis 25 % liegt.

3. Verfahren nach Anspruch 1 oder 2, dadurch gekennzeichnet, daß man die die zu bestimmende freie Substanz enthaltende biologische Flüssigkeit im wesentlichen gleichzeitig mit dem markierten Antikörper und der an die feste Phase gebundenen Substanz oder deren Derivat in Kontakt bringt und gemeinsam inkubiert.

4. Verfahren nach einem der Ansprüche 1 bis 3, dadurch gekennzeichnet, daß die zu bestimmende freie Substanz Thyroxin (FT₄) oder Trijodthyronin (FT₃) ist.

5. Verfahren nach Anspruch 4, dadurch gekennzeichnet, daß die zu bestimmende Substanz FT₃ oder FT₄ ist und der verwendete Antikörper ein Anti-T₄- oder Anti-T₃-Antikörper mit einer Affinitätskonstanten von 10¹⁰ l/mol oder weniger ist.

6. Verfahren nach einem der Ansprüche 1 bis 5, dadurch gekennzeichnet, daß die feste Phase ein Kunststoff oder Glas ist.

7. Verfahren nach einem der Ansprüche 1 bis 6, dadurch gekennzeichnet, daß die an die feste Phase gebundene Substanz oder deren Derivat an eine Trägerkomponente gebunden in Form eines Konjugats vorliegt.

8. Verfahren nach Anspruch 7, dadurch gekennzeichnet, daß die Trägerkomponente ein Protein, Polypeptid oder ein Polysaccharid ist.

9. Verfahren nach Anspruch 7 oder 8, dadurch gekennzeichnet, daß der markierte Antikörper bzw. das markierte Antikörpergemisch gegenüber der Trägerkomponente eine Kreuzreaktivität von weniger als 0,5 % aufweist.

## Claims

1. Immunoassay for the determination of free substances exhibiting haptene properties, especially the determination of hormones, steroids, drugs or their metabolites, and vitamins or toxins, in biological fluids in the presence of one or more physiological bonding partners for the substance to be determined, wherein the biological fluid is reacted with
(a) a defined amount of an antibody which is specific against the substance to be determined, or an antibody mixture with a detectable marking component, and
(b) an excess of the substance to be determined or a derivative of this substance in an immobilised form due to bonding with a solid phase,
and wherein the solid phase is afterwards separated from the liquid phase and the content of the marked antibody in the liquid and/or solid phase is determined by measurement of the marking, and then the content of the free substance to be determined in the biological specimen is obtained, with the use of a calibration curve, by calculation from the results of the measurements made, characterised in that, the immobilised form of the substance or its derivative is so chosen that the affinity of the specifically marked antibody or antibody mixture towards the immobilised form amounts to less than 28% of the affinity of this antibody or antibody mixture towards the substance whose free amount is to be determined in the biological liquid (''cross reactivity < 28%).

2. Process in accordance with Claim 1, characterised in that, the cross reactivity is in the region from 8 to 25%.

3. Process in accordance with Claim 1 or 2, characterised in that, the biological liquid containing the free substance to be determined is brought into contact essentially at the same time with the marked antibody and with the substance or its derivative bonded to the solid phase, and they are incubated together.

4. Process in accordance with one of the Claims 1 to 3, characterised in that, the free substance to be determined is L-thyroxine (FT₄) or L-triiodothyronine (FT₃).

5. Process in accordance with Claim 4, characterised in that, the substance to be determined is FT₄ or FT₃ and the antibody used is an anti-T₄-antibody or an anti-T₃-antibody with an affinity constant of 10¹⁰ l/mol or less.

6. Process in accordance with one of the Claims 1 to 5, characterised in that, the solid phase is a plastic or glass.

7. Process in accordance with one of the Claims 1 to 6, characterised in that, the substance or its derivative bonded to the solid phase occurs in the form of a conjugate bonded to a carrier component.

8. Process in accordance with Claim 7, characterised in that, the carrier component is a protein, a polypeptide or a polysaccharide.

9. Process in accordance with Claim 7 or 8, characterised in that, the marked antibody or the marked antibody mixture respectively exhibits a cross reactivity of less than 0.5% towards the carrier component.

## Revendications

1. Méthode de dosage immunologique pour la détection de substances libres douées de propriétés hapténiques, notamment d'hormones, de stéroïdes, de drogues ou de leurs métabolites, de vitamines ou de toxines, dans des liquides biologiques en présence d'un ou plusieurs partenaires physiologiques de liaison pour les substances à doser, dans laquelle le liquide biologique est amené à réagir avec
a) une quantité définie d'un anticorps spécifique dirigé contre la substance à doser ou d'un mélange d'anticorps avec un constituant de marquage pouvant être détecté et
b) un excès de la substance à doser ou d'un dérivé de cette substance sous une forme immobilisée par liaison à une phase solide,
et dans laquelle on sépare ensuite la phase solide de la phase liquide et on détermine la teneur en anticorps marqué de la phase liquide et/ou de la phase solide par mesure du marquage puis on détermine par interprétation par le calcul des résultats de mesure obtenus la teneur de l'échantillon biologique en substance libre à doser, en utilisant des courbes d'étalonnage,
caractérisée en ce que la forme immobilisée de la substance ou de son dérivé est choisie de manière que l'affinité de l'anticorps marqué spécifique ou du mélange d'anticorps vis-à-vis de la forme immobilisée s'élève à moins de 28% de l'affinité de cet anticorps ou de ce mélange d'anticorps vis-à-vis de la substance dont on se propose de doser la fraction libre dans le liquide biologique ("réactivité croisée < 28%").

2. Méthode suivant la revendication 1, caractérisée en ce que la réactivité croisée se situe dans l'intervalle de 8 à 25%.

3. Méthode suivant la revendication 1 ou 2, caractérisée en ce qu'on met en contact et on fait incuber ensemble le liquide biologique contenant la substance libre à doser essentiellement en même temps que l'anticorps marqué et la substance liée à la phase solide ou son dérivé.

4. Méthode suivant l'une des revendications 1 à 3, caractérisée en ce que la substance libre à doser est la L-thyroxine (FT₄) ou la L-triiodothyronine (FT₃).

5. Méthode suivant la revendication 4, caractérisée en ce que la substance à doser est FT₄ où FT₃ et l'anticorps utilisé est un anticorps anti-T₄ ou anti-T₃ ayant une constante d'affinités égale ou inférieure à 10¹⁰ l/mole.

6. Méthode suivant l'une des revendications 1 à 5, caractérisée en ce que la phase solide est une matière plastique ou du verre.

7. Méthode suivant l'une des revendications 1 à 6, caractérisée en ce que la substance ou son dérivé lié à la phase solide se présente en liaison avec un composant de support sous forme d'un conjugué.

8. Méthode suivant la revendication 7, caractérisée en ce que le composant de support est une protéine, un polypeptide ou un polysaccharide.

9. Méthode suivant la revendication 7 ou 8, caractérisée en ce que l'anticorps marqué ou le mélange d'anticorps marqués présente vis-à-vis du composant de support une réactivité croisée de moins de 0,5%.
